# EUROPEAN PATENT SPECIFICATION

(11) **EP 1 227 088 B1**
(45) Date of publication and mention of the grant of the patent: **17.09.2003**
(21) Application number: 02009350.6
(22) Date of filing: 28.02.2001
(51) Int. Cl.: C07D 307/87, A61K 31/343, A61P 25/24

(54) **Crystalline base of citalopram and hydrochoride or hydrobromide salt thereof**
Kristalline Base von Citalopram, und Hydrochlorid- oder Hydrobromidsalz davon
Base crystalline de citalopram et son sel hydrochlorique ou hydrobromique

(30) Priority: 13.03.2000 DK 200000402; 13.04.2000 WO PCT/DK00/00183
(43) Date of publication of application: 31.07.2002
(62) Divisional of application: 01909568.6
(73) Proprietor: H.Lundbeck A/S, 2500 Valby-Copenhagen (DK)
(72) Inventor: Petersen, Hans, 2720 Vanlöse (DK); Bögesö, Klaus Peter, 2970 Hörsholm (DK); Holm, Per, 2720 Vanlöse (DK)
(74) Representative: HOFFMANN - EITLE

(56) References cited:
- EP-A- 0 171 943
- EP-A- 0 347 066
- WO-A-98/19511
- WO-A-98/19512
- WO-A-98/19513
- DE-A- 2 657 013
- DE-U- 20 007 303

## Description

The present invention relates to the crystalline base of the well known antidepressant drug citalopram,1-[3-(dimethylamino)propyl]-1-(4-fluorophenyl)-1,3-dihydro-5-isobenzofurancarbonitrile and formulations of said base.

### Background of the Invention

Citalopram is a well-known antidepressant drug that has now been on the market for some years and has the following structure:

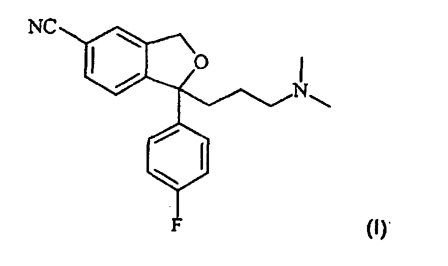

It is a selective, centrally acting serotonin (5-hydroxytryptamine; 5-HT) reuptake inhibitor, accordingly having antidepressant activities. The antidepressant activity of the compound has been reported in several publications, eg. J. Hyttel, *Prog. Neuro-Psychopharmacol. & Biol*. *Psychiat*., **1982**, *6*, 277-295 and A. Gravem, *Acta Psychiatr. Scand*., **1987**, *75* , 478-486. The compound has further been disclosed to show effects in the treatment of dementia and cerebrovascular disorders, EP-A-474580.

Citalopram was first disclosed in DE 2,657,013, corresponding to US 4,136,193. This patent publication describes the preparation of citalopram by one method and outlines a further method, which may be used for preparing citalopram. The citalopram prepared was isolated as the oxalate, the hydrobromide and the hydrochloride salt, respectively. Furthermore, the citalopram base was obtained as an oil (B.P. 175 C/0.03 mmHg). Citalopram is marketed as the hydrobromide and the hydrochloride, respectively.

A number of processes for the preparation of citalopram have been disclosed. In many of these, the last step of the process is a conversion of a group different from cyano in the 5 position of the direct analogue of citalopram to a 5-cyano group. So citalopram has been prepared by:
Exchange of 5-halogen, or 5-CF₃-(CF₂)ₙ-SO₂-O- with cyano (DE 2,657,013 and co-pending WO 0011926 and WO 0013648)
Conversion of a 5-amido or 5-ester group to a 5-cyano group (WO 9819513)
Conversion of a 5-amino group to a 5-cyano group (WO 9819512)
Conversion of a 5-formyl group to a 5-cyano group (WO 9900548)
Conversion of a 5-oxazolinyl or 5-thiazolinyl group to a 5-cyano group (WO 0023431)

Other processes for the preparation of citalopram comprise exchange of the 5-bromo group of 1-(4-fluorophenyl)-1,3-dihydro-5-isobenzofuranbromide with 5-cyano followed by alkylation with a 3-(N,N-dimethylamino)propyl-halogenide (DE 2,657,013 and WO 9819511).

Many of the processes mentioned above have the disadvantage that it is difficult to separate the intermediates formed during the process (the intermediates mentioned above or earlier intermediates) from the end product and, accordingly, extensive purification procedures involving loss of citalopram are required in order to obtain the necessary quality of the end product.

It has now been found that the base of citalopram may be obtained as a very nice and pure crystalline product, which may easily be handled and conveniently be formulated into tablets and other pharmaceutical forms. Furthermore, it has surprisingly been found that a very good and efficient purification of citalopram may be obtained during manufacture of citalopram (e.g. of the hydrobromide or the hydrochloride salt) by crystallising the base, and thereafter optionally forming a salt from the base.

This purification process is particularly useful for removing intermediates which are structurally closely related to citalopram, in particular compounds which only differ from citalopram by the substituent situated in position 5 on the isobenzofurane ring, and intermediates which have physical/chemical properties which are close to those of citalopram, e.g. the 1-[3-(dimethylamino)propyl]-1-(4-fluorophenyl)-1,3-dihydro-isobenzofuranes having halogen (in particular bromide and chloride), an amide or an ester in position 5 of the isobenzofurane ring, or 1-(4-fluorophenyl)-1,3-dihydro-5-isobenzofuranbromide, or -cloride.

### Summary of the invention

The present invention provides the crystalline base of the compound

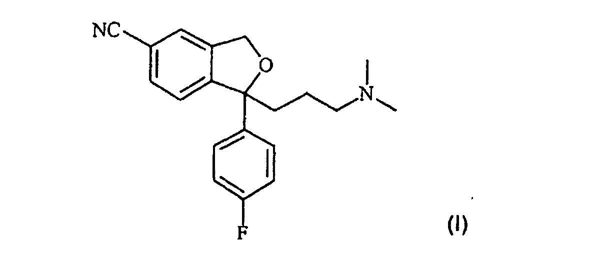

More particularly, the present invention relates to a process for the manufacture of citalopram base characterised in that one or more impurities of the formula 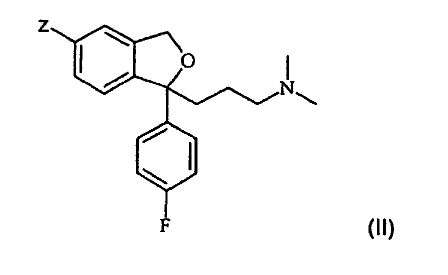 wherein Z is halogen, -O-SO₂-(CF₂)ₙ-CF₃, where n is 0-8, -CHO, -NHR¹, -COOR², -CONR²R³ wherein R² and R³ are selected from hydrogen, alkyl, optionally substituted aryl or aralkyl and R¹ is hydrogen or alkylcarbonyl, are removed from a crude mixture of citalopram or from a crude salt of citalopram, by precipitating citalopram base in crystalline form, and optionally re-crystallising said base one or more times.

The crude mixture of citalopram containing the compound of formula II as an impurity may be prepared by subjecting a compound of formula II to a cyanide exchange reaction with a cyanide source, or by subjecting 1-(4-fluorophenyl)-1,3-dihydro-5-isobenzofuranhalogenide, in particular the bromide, to a cyanide exchange reaction followed by alkylation with a 3-(N,N-dimethylamino)propyl-halogenide.

In a particular embodiment of the invention, Z is halogen, in particular bromide or chloride.

The crude salt may be any convenient salt, such as the hydrobromide, hydrochloride, sulphate, oxalate, phosphate, nitrate or any other convenient salt. Other salts are salts of organic acids.

In a preferred embodiment of the invention, the crude salt is the sulphate, the hydrobromide or the hydrochloride salt.

In yet another aspect, a pharmaceutical formulation of the free base of citalopram is provided. Preferably the formulation is for oral administration.

The formulations according to the invention may be prepared by direct compression of citalopram in admixture with conventional adjuvants or diluents. Alternatively, a wet granulate or a melt granulate of citalopram, optionally in admixture with conventional adjuvants or diluents may be used for compression of tablets.

In particular, the pharmaceutical composition of the invention contains the racemic mixture of citalopram base.

The crystalline base of citalopram is preferably more than 99.8% w/w pure, most preferably more than 99.9% w/w pure (peak area). The melting point is preferably a range within 90 - 93 °C, most preferably 91 - 92 °C (DSC; onset, open capsule) or it is between 92 and 94°C, preferably 92.5 and 93.5 °C (DSC; onset, closed capsule). The crystalline base of citalopram is preferably in racemic form.

The terms "crude salt" and "crude mixture" refer to the fact that the salt and the mixture, respectively, comprise impurities, in particular impurities of formula II, which must be removed or which it is desired to remove.

The crude salt may be a salt separated directly from the reaction mixture, or the crude reaction mixture may have been subjected to some initial purification, e.g. one re-crystallisation, and/or treatment with activated carbon or silica gel, and the salt formed subsequently by treatment with an acid using methods known in the art. The salt may be isolated by precipitation or it may exist in a solvent, e.g. in the mixture resulting directly from the synthesis of the salt.

Similarly, the crude mixture comprising citalopram may be obtained directly from the synthesis of the compound according to any of the above mentioned processes or it may have been subjected to some initial or simultaneous purification, e.g. one re-crystallisation, treatment with activated carbon or silica gel.

The base of citalopram may be set free from the crude salt by dissolving the crude salt in a mixture of water and an organic solvent and then adding a base. The organic solvent may be toluene, ethyl acetate or any other suitable solvent and the base may be any convenient base, preferably NaOH or NH₃. Likewise, the base of citalopram may, if necessary, be set free from a crude mixture containing citalopram by treatment with a base.

Crude mixtures containing citalopram base may be subjected to further purification and extraction, before the base is precipitated in crystalline form. The base of citalopram may be isolated by separation of the organic phase, evaporation of the solvent in order to obtain the base most probably as an oil and then crystallisation of the base from an aprotic solvent, such as an alkane, including n-heptane, hexane and isooctane, and high and low boiling petroleum ethers and substituted aromates, incI toluene and xylenes. Crystalline citalopram base may be re-crystallised from the same solvents.

The cyanide exchange reactions mentioned above may be carried out as described in the patent applications mentioned above.

In particular, when Z is halogen, or CF₃-(CF₂)ₙ-SO₂-O- wherein n is an integer in the range 0-8, incl., the conversion to a cyano group may be carried out by reaction with a cyanide source, for example KCN, NaCN, CuCN, Zn(CN)₂ or (R⁴)₄NCN where R⁴ indicates four groups which may be the same or different and are selected from hydrogen and straight chain or branched alkyl, in the presence of a palladium catalyst and a catalytic amount of Cu⁺ or Zn²⁺, or with Zn(CN)₂ in the presence of a palladium catalyst.

The cyanide source is used in a stoichiometric amount or in excess, preferably 1-2 equivalents are used pr. equivalent starting material. (R⁴)₄N⁺ may conveniently be (Bu)₄N⁺. The cyanide compound is preferably NaCN or KCN or Zn(CN)₂.

The palladium catalyst may be any suitable Pd(0) or Pd(II) containing catalyst, such as Pd(PPh₃)₄, Pd₂(dba)₃, Pd(PPh)₂Cl₂, etc. The Pd catalyst is conveniently used in an amount of 1-10, preferably 2-6, most preferably about 4-5 mol%.

Catalytic amounts of Cu⁺ and Zn²⁺, respectively, means substoichiometric amounts such as 0.1- 5, preferably 1 - 3 %. Conveniently, about ½ eq. is used per eq. Pd. Any convenient source of Cu⁺ and Zn⁺⁺ may be used. Cu⁺ is preferably used in the form of CuI and Zn²⁺ is conveniently used as the Zn(CN)₂ salt

When Z is Br or I, the conversion to a cyano group may also be carried out by reaction with Cu(CN) without catalyst. In a preferred embodiment, the reaction is performed at elevated temperature.

In another aspect the reaction is performed in an ionic liquid of the general formula (R⁵)₄N⁺, X⁻, wherein R⁵ are alkyl-groups or two of the R⁵groups together form a ring and X⁻ is the counterion. In one embodiment, (R⁵)₄N⁺X⁻ represents

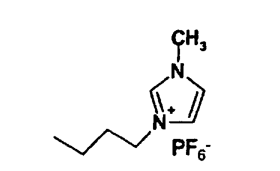

In another particular aspect, the reaction is conducted with apolar solvents such as benzene, xylene or mesitylene and under the influence of microwaves by using *i.e.* Synthewave 1000™ by Prolabo. In a particular aspect, the reaction is performed without added solvent.

The temperature ranges are dependent upon the reaction type. If no catalyst is present, preferred temperatures are in the range of 100-200°C. However, when the reaction is conducted under the influence of microwaves, the temperature in the reaction mixture may raise to above 300 °C. More preferred temperature ranges are between 120-170°C. The most preferred range is 130-150°C. If catalyst is present, the preferred temperature range is between 0 and 100°C. More preferred are temperature ranges of 40-90°C. Most preferred temperature ranges are between 60-90°C.

Other reaction conditions, solvents, etc. are conventional conditions for such reactions and may easily be determined by a person skilled in the art.

When Z is Cl or Br, the conversion to a cyano group may also be carried out by reaction with a cyanide source, for example KCN, NaCN, CuCN, Zn(CN)₂ or (R⁴)₄NCNwhere (R⁴)₄ indicates four groups which may be the same or different and are selected from hydrogen and straight chain or branched alkyl, in the presence of a nickel catalyst.

The nickel catalyst may be any suitable Ni(0) or Ni(II) containing complex which acts as a catalyst, such as Ni(PPh₃)₃, (σ-aryl)-Ni(PPh₃)₂Cl, etc. The nickel catalysts and their preparation are described in WO 96/11906, EP-A-613720 or EP-A-384392.

In one embodiment, the reaction is carried out in the presence of a catalytic amount of Cu⁺ or Zn²⁺.

In a particularly preferred embodiment, a Nickel(0) complex is prepared*in situ* before the cyanation reaction by reduction of a Nickel(II) precursor such as NiCl₂ or NiBr₂ by a metal, such as zinc, magnesium or mangan in the presence of excess of complex ligands, preferably triphenylphosphin.

The Ni-catalyst is conveniently used in an amount of 0.5-10, preferably 2-6, most preferably about 4-5 mol%. Catalytic amounts of Cu⁺ and Zn²⁺, respectively, mean substoichiometric amounts such as 0.1- 5, preferably 1 - 3 %. Any convenient source of Cu⁺ and Zn²⁺ may be used. Cu⁺ is preferably used in the form of CuI and Zn²⁺ is conveniently used as the Zn(CN)₂ salt or formed in situ by reduction of a Nickel (II) compounds using zinc.

The Ni catalysts are *i*.*e*. Ni (0), Pd(0) or Pd(II) catalysts as described by Sakakibara et. al. in Bull. Chem. Soc. Jpn., 61, 1985-1990, (1988). Preferred catalysts arc Ni(PPh₃)₃ or Pd(PPh₃)₄, or Pd(PPh)₂Cl₂.

The reactions may be performed in any convenient solvent as described in Sakakibara et. al. in Bull. Chem. Soc. Jpn., 61, 1985-1990, (1988). Preferred solvents are acetonitril, ethylacetat, THF, DMF or NMP.

When Z is CHO, the conversion to a cyano group may be carried out by conversion of the formyl group to an oxime or similar group by reaction with a reagent R⁶-V-NH₂ wherein R⁶ is hydrogen, optionally substituted alkyl, aryl or heteroaryl and V is O, N or S, followed by dehydration with a common dehydrating agent, for example thionylchloride, acetic anhydride/pyridine, pyridine/HCl or phosphor pentachloride. Preferred reagents R⁶-V-NH₂ are hydroxylamin and compounds wherein R⁶ is alkyl or aryl and V is N or O.

When Z is -COOH, the conversion to a cyano group may be carried out via the corresponding acid chloride, ester or amide.

The acid chloride is conveniently obtained by treatment of the acid with POCl₃, PCl₅ or SOCl₂ neat or in a suitable solvent, such as toluene or toluene comprising a catalytic amount of N,N-dimethylformamide. The ester is obtained by treatment of the acid with an alcohol, in the presence of an acid. preferably a mineral acid or a Lewis acid, such as HCl, H₂SO_{4,} POCl₃, PCl₅ or SOCl₂. Alternatively, the ester may be obtained from the acid chloride by reaction with an alcohol. The ester or the acid chloride is then converted to an amide by amidation with ammonia or an alkylamine, preferably t-butyl amine.

The conversion to amide may also be obtained by reaction of the ester with ammonia or an alkylamine under pressure and heating.

The amide group is then converted to a cyano group by dehydration. The dehydrating agent may be any suitable dehydrating agent, and the optimal agent may easily be determined by a person skilled in the art. Examples of suitable dehydrating agents are SOCl₂, POCl₃ and PCl₅, preferably SOCl₂.

In a particularly preferred embodiment, the carboxylic acid is reacted with an alcohol, preferably ethanol, in the presence of POCl₃, in order to obtain the corresponding ester, which is then reacted with ammonia thereby giving the corresponding amide, which in turn is reacted with SOCl₂ in toluene comprising a catalytic amount of N,N-dimethylformamide.

Alternatively, a compound where Z is -COOH may be reacted with chlorosulfonyl isocyanate in order to form the nitrile, or treated with a dehydrating agent and a sulfonamide.

When Z is -NHR¹, where R¹ is hydrogen, the conversion into cyano is preferably performed by diazotation and followed by reaction with CN⁻. Most preferably NaNO₂ and CuCN and/or NaCN are used. When R¹ is alkylcarbonyl, it is initially subjected to hydrolysis thereby obtaining the corresponding compound wherein R¹ is H which is the converted as described above. The hydrolysis may be performed either in acidic or basic environment.

The compounds of formula (II) may be prepared as described in DE 2,657,013, WO 0011926 and WO 0013648, WO 9819513, WO 9819512 and WO 9900548.

Throughout this specification with claims halogen means chloro, bromo or iodo.

The term alkyl refers to a branched or unbranched alkyl group, such as methyl, ethyl, 1-propyl, 2-propyl, 1-butyl, 2-butyl, 2-methyl-2-propyl, and 2-methyl-1-propyl.

The term aryl refers to a carbocyclic aromatic group, in particular phenyl. Aralkyl refers to an arylalkyl group wherein aryl and alkyl is as defined above. The aryl and aralkyl groups may optionally be substituted, e.g. with alkyl groups, forming for example tolyl.

The pharmaceutical compositions of the invention may be administered in any suitable way and in any suitable form, for example orally in the form of tablets, capsules, powders or syrups, or parenterally in the form of usual sterile solutions for injection. Preferably the pharmaceutical compositions of the invention are administered orally.

The pharmaceutical formulations of the invention may be prepared by conventional methods in the art. For example, tablets may be prepared by mixing the active ingredient with ordinary adjuvants and/or diluents and subsequently compressing the mixture in a conventional tabletting machine. Examples of adjuvants or diluents comprise: Corn starch, potato starch, talcum, magnesium stearate, gelatine, lactose, gums, and the like. Any other adjuvant or additive, colourings, aroma, preservatives etc. may be used provided that they arc compatible with the active ingredients.

In particular, the formulations according to the invention may be prepared by direct compression of citalopram in admixture with conventional adjuvants or diluents. Alternatively, a wet granulate or a melt granulate of citalopram, optionally in admixture with conventional adjuvants or diluents may be used for compression of tablets.

Solutions for injections may be prepared by solving the active ingredient and possible additives in a part of the solvent for injection, preferably sterile water, adjusting the solution to the desired volume, sterilisation of the solution and filling in suitable ampoules or vials. Any suitable additive conventionally used in the art may be added, such as tonicity agents, preservatives, antioxidants, etc.

According to the present invention, the base of citalopram has been found to be crystalline with stable and nice white crystals and it has been found that the base may easily be crystallised in a very pure form. So for example more than 99.8% w/w pure citalopram base was obtained by crystallisation from up to 95% pure hydrobromide without further purification. Accordingly, the yield may be improved substantially during the manufacture of citalopram.

Finally, it has been found that the crystalline citalopram base may be formulated into very good and stable solid formulations with good release properties.

The invention is further illustrated by the following examples.

### Example 1

### Crystallisation of R,S-Citalopram as the free base.

1-(3-Dimethylaminopropyl)-1-(4'-fluorophenyl)-1,3-dihydrobenzofuran-5-carbonitrile.

1-(3-Dimethylaminopropyl)-1-(4'-fluorophenyl)-1,3-dihydrobenzofuran-5-carbonitrile hydrobromide (101 grams, 0.25 mole) prepared from 1-(3-Dimethylaminopropyl)- 1-(4-fluorophenyl)-1,3-dihydro-5-isobenzofuranbromide, is suspended in water (500 ml) and toluene (500 ml). NaOH (60 ml, 5 N (aq)) is added and the mixture (pH>10) is stirred for 15 min. before the phases are separated. The organic phase is washed with water (2x100 ml) and filtered through a pad of filter help. The volatiles are removed *in vacuo* and the title compound is obtained as an oil. n-Heptane (400 ml) is added and the mixture is heated to 70 °C. On cooling, crystals form. The white crystals of the title compound are filtered off and dried at ambient temperature over night in vacuo. Yield: 75.4 grams (93%). DSC(onset, open capsule): 91.3-91.8 °C DSC (onset, closed capsule): 92.8 °C. Purity: (> 99.8 % (peak area)). Anal. calcd. for C20H21N2F1O1; C, 74.04; H, 6.54; N, 8.64. Found C, 74.01; H, 6.49; N, 8.59. 1H-NMR (DMSO-d6 , 500 MHz): 1.21 (1H, m), 1.29 (1H, m), 2.02 (6H, s), 2.09-2.23 (4 H, m), 5.15 (1H, d J=12.5 Hz), 5.22 (1H, d J=12.5 Hz), 7.16 (2H, t J=8.5 Hz), 7.60 (2H, dt J=8.5 Hz J=1.2 Hz), 7.76 (1H, d J= 8.5 Hz), 7.79 (1H, d J=8.5 Hz), 7.80 (1H, s). 13C-NMR (DMSO-d6, 125 MHz): 21.8, 38.3, 45.0, 58.8, 71.0, 90.7, 110.5, 115.1 (d J=22 Hz), 118.8, 123.1, 125.1, 127.0 (d J=8 Hz), 132.0, 140.0 (d J=3 Hz), 140.5, 149.5, 161.3 (d J=245Hz).

### Example 2

a) A crude mixture of Citalopram and sulphuric acid is made basic by adding NaOH and the citalopram base is extracted with toluene. The toluene is evaporated and the citalopram base obtained is dissolved in n-heptane at elevated temperature. The very pure free base of citalopram is precipitated by cooling.
b) A crude mixture of Citalopram and sulphuric acid is made basic by adding NaOH and the citalopram base is extracted with toluene. The toluene is evaporated and the citalopram base obtained is dissolved in methanol. The mixture is treated with activated carbon and filtrated and the solvent is evaporated. The purified free base is dissolved in n-heptane at elevated temperature. Then the very pure free base of citalopram is precipitated by cooling.
c) A crude mixture of Citalopram and sulphuric acid is made basic by adding NaOH and the citalopram base is extracted with toluene. The toluene phase is treated with silicagel, the toluene is evaporated and the citalopram base obtained is dissolved in n-heptane at elevated temperature. The very pure free base of citalopram is precipitated by cooling.
d) A crude mixture of Citalopram and sulphuric acid is made basic by adding NaOH and the citalopram base is extracted with toluene. The toluene phase is treated with silicagel, the toluene is evaporated and the citalopram base obtained is dissolved in methanol. The mixture is treated with activated carbon and filtrated and the solvent is evaporated. The purified free base is dissolved in n-heptane at elevated temperature. Then the extremely pure free base of citalopram is precipitated by cooling.

### Example 3

### Wet granulation and preparation of tablets

The batch size was 200 g and the granulation was performed in a small-scale laboratory high shear mixer (Micromixer).

Citalopram base was sieved through a sieve aperture of 0.3 mm. The ingredients of the intragranular phase (1 - 4 in Table 1) were mixed at 600 rpm. 25 ml of purified water (5) was added in 30 sec and the granulation terminated after a total processing time of 3 min. The granulate was wet sieved through a 0.7 mm sieve aperture and dried at 40 °C in 30 minutes to equilibrium relative humidity of 32 %. The dried granulate was finally sieved through a 0.7 mm sieve aperture.

The dried granulate was mixed for 3 minutes with the extragranular phase (6 - 7) in a Turbula mixer and finally mixed with the lubricant (8) for 30 sec.

**Table 1.**

| Composition of the tablets. | | |
|---|---|---|
| | Materials | % |
| 1 | Citalopram (base) | 16.00 |
| 2 | Kollidon VA64 | 2.32 |
| 3 | Lactose 350 mesh | 38.98 |
| 4 | Corn starch | 20.00 |
| 5 | Purified water | 25 |
| 6 | Avicel PH 200 (Microcrystalline cellulose) | 20.00 |
| 7 | Ac-Di-Sol (Croscarmelose sodium) | 2.00 |
| 8 | Magnesium stearate | 0.7 |

Tablets were produced on a single punch tabletting machine Korsch EK0. The characteristics of the tables are shown in Table 2.

**Table 2.**

| Tablet characteristics. | |
|---|---|
| Parameter | Values |
| Tablet strength, mg | 20 |
| Nominal tablet weight, mg | 125 |
| Tablet diameter, mm | 7 |
| Tablet shape | Film coating (special doomed) |
| Mean disintegration time, min | 1.77 |
| Mean chrushing strength, N | 69.1 |
| Mean tablet weight, mg | 125.4 |
| RSD tablet weight, % | 0.42 |
| Friability, % | 0.3 |

The tablets produced had satisfactory technical properties.

### Example 4

### Melt granulation

The batch size was 200 g. Citalopram base was sieved through a sieve aperture of 0.3 mm. The granulation was performed in a small-scale laboratory high shear mixer (Micromixer)

The ingredients of the intra-granular phase (1 - 3 in Table 3) were mixed at 1200 rpm.
The jacket temperature was 80 °C. The granulation process was terminated after 3.5 min. The granulate was sieved through a sieve aperture of 1.0 mm and mixed with the extra-granular phase (4, 5) for 3 min. and with the lubricant (6) for 30 sec.

**Table 3.**

| Composition of the tablet. | | |
|---|---|---|
| | Materials | % |
| 1 | Citalopram (base) | 16.00 |
| 2 | Polyethyleneglycol 6000 | 9.14 |
| 3 | Lactose 350 mesh | 38.98 |
| 4 | Avicel PH 200 (Microcrystalline cellulose) | 30.00 |
| 5 | Kollidon CL (Cross-linked povidone) | 4.00 |
| 6 | Magnesium stearate | 0.7 |

Tablets were produced on a single punch tabletting machine Korsch EK0. The characteristics of the tables are shown in Table 4.

**Table 4.**

| Tablet characteristics. | |
|---|---|
| Parameter | Values |
| Tablet strength, 20 mg | 20 |
| Nominel tablet weight, mg | 125 |
| Tablet diameter, mm | 7 |
| Tablet shape | Film coating , Special doomed |
| Mean disintegration time, min | 1.0 |
| Mean chrushing strength, N | 55.5 |
| Mean tablet weight, mg | 125.6 |
| RSD tablet weight, % | 0.5 |
| Friability, % | 0.4 |

The tablets produced had satisfactory technical properties.

## Claims

1. Crystalline base of citalopram, **characterised in that** it has a purity of more than 99.8 %w/w, preferably more than 99.9 % w/w.

2. Crystalline base of citalopram, prepared by a process **characterised in that** the base of citalopram is set free and precipitated in crystalline form and optionally re-crystallised one or more times.

3. The crystalline base of citalopram according to claim 2 **characterised in that** the base of citalopram is set free from a crude salt or a crude mixture of citalopram.

4. Crystalline base of citalopram, prepared by a process which is **characterised in that** one or more impurities of the formula 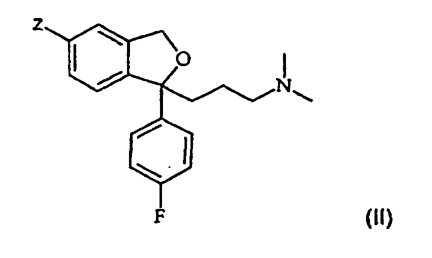 wherein Z is halogen, -O-SO₂-(CF₂)ₙ-CF₃, where n is 0-8, -CHO, -NHR¹ , -COOR², -CONR²R³ wherein R² and R³ are selected from hydrogen, alkyl, optionally substituted aryl or aralkyl and R¹ is hydrogen or alkylcarbonyl, are removed from a crude mixture of citalopram or from a crude salt of citalopram, by precipitating citalopram base in crystalline form and optionally re-crystallising said base one or more times.

5. The crystalline base of citalopram according to claim 4 wherein the crude mixture of citalopram containing the compound of formula II as an impurity, is prepared by subjecting a compound of formula II to a cyanide exchange reaction with a cyanide source.

6. The crystalline base of citalopram according to claim 4 wherein Z is halogen, in particular bromide or chloride.

7. The crystalline base of citalopram according to any one of claims 4 to 6 wherein the crude mixture of citalopram is subjected to initial purification before the base of citalopram is precipitated in crystalline form.

8. The crystalline base of citalopram according to any one of claims 4 to 6 wherein the crude mixture of citalopram is subjected to initial purification before a crude salt is formed from said crude mixture.

9. The crystalline base of citalopram according to any one of claims 4 to 7 wherein the base of citalopram is set free from a crude salt or a crude mixture of citalopram by treatment with a base and optionally subjected to further purification before the base of citalopram is precipitated in crystalline form.

10. The crystalline base of citalopram according to any one of claims 2 to 4, **characterised in that** the crude salt is a hydrobromide, hydrochloride, sulphate, oxalate, phosphate or nitrate salt, preferably the sulphate, hydrobromide, or hydrochloride salt.

11. The crystalline base of citalopram according to any one of claims 2 to 10, **characterised in that** it has a purity of more than 99.8 %w/w, preferably more than 99.9 %w/w.

12. A pharmaceutical composition containing the crystalline base of citalopram according to any one of claims 1 to 11.

13. The pharmaceutical composition according to claim 12 which is a tablet prepared by
a) direct compression of citalopram, optionally in admixture with pharmaceutically acceptable adjuvants;
b) compression of a wet granulate of the citalopram, optionally in admixture with pharmaceutically acceptable adjuvants; or
c) compression of a melt granulate of the citalopram, optionally in admixture with pharmaceutically acceptable adjuvants.

14. The pharmaceutical composition according to claim 12 or 13, **characterized in that** it contains the racemic mixture of citalopram base.

## Patentansprüche

1. Kristalline Base von Citalopram, **dadurch gekennzeichnet, daß** sie eine Reinheit von mehr als 99,8 % G/G, bevorzugt von mehr als 99,9 % G/G hat.

2. Kristalline Base von Citalopram, hergestellt durch ein Verfahren, das **dadurch gekennzeichnet ist, daß** die Base von Citalopram freigesetzt und in kristalliner Form ausgefällt und ggf. ein- oder mehrmals umkristallisiert wird.

3. Kristalline Base von Citalopram gemäß Anspruch 2, **dadurch gekennzeichnet, daß** die Base von Citalopram aus einem rohen Salz oder einer rohen Mischung von Citalopram freigesetzt wird.

4. Kristalline Base von Citalopram, hergestellt durch ein Verfahren, das **dadurch gekennzeichnet ist, daß** eine oder mehrere Verunreinigungen der Formel 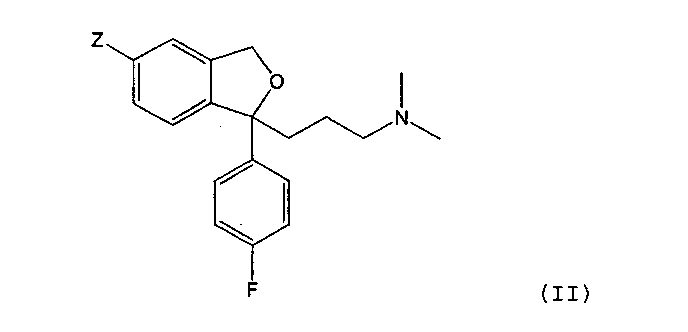 worin Z Halogen, -O-SO₂-(CF₂)n-CF₃, worin n 0-8 ist, -CHO, -NHR¹, -COOR², -CONR²R³ ist, worin R² und R³ aus Wasserstoff, Alkyl, ggf. substituiertem Aryl oder Aralkyl ausgewählt sind und R¹ Wasserstoff oder Alkylcarbonyl ist, aus einer rohen Mischung von Citalopram oder aus einem rohen Salz von Citalopram entfernt werden, indem Citalopram-Base in kristalliner Form ausgefällt und die Base ggf. ein- oder mehrmals umkristallisiert wird.

5. Kristalline Base von Citalopram gemäß Anspruch 4, worin die rohe Mischung von Citalopram, die die Verbindung der Formel (II) als Verunreinigung enthält, hergestellt wird durch Unterwerfen einer Verbindung der Formel (II) einer Cyanidaustauschreaktion mit einer Cyanidquelle.

6. Kristalline Base von Citalopram gemäß Anspruch 4, worin Z Halogen ist, insbesondere Bromid oder Chlorid.

7. Kristalline Base von Citalopram gemäß einem der Ansprüche 4 bis 6, worin die rohe Mischung von Citalopram einer anfänglichen Reinigung unterworfen wird, bevor die Base von Citalopram in kristalliner Form ausgefällt wird.

8. Kristalline Base von Citalopram gemäß einem der Ansprüche 4 bis 6, worin die rohe Mischung von Citalopram einer anfänglichen Reinigung unterworfen wird, bevor ein rohes Salz aus der rohen Mischung gebildet wird.

9. Kristalline Base von Citalopram gemäß einem der Ansprüche 4 bis 7, worin die Base von Citalopram aus einem rohen Salz oder einer rohen Mischung von Citalopram durch Behandlung mit einer Base freigesetzt und ggf. einer weiteren Reinigung unterworfen wird, bevor die Base von Citalopram in kristalliner Form ausgefällt wird.

10. Kristalline Base von Citalopram gemäß einem der Ansprüche 2 bis 4, **dadurch gekennzeichnet, daß** das rohe Salz ein Hydrobromid-, Hydrochlorid-, Sulfat-, Oxalat-, Phosphat- oder Nitratsalz ist, bevorzugt das Sulfat-, Hydrobromid- oder Hydrochloridsalz.

11. Kristalline Base gemäß einem der Ansprüche 2 bis 10, **dadurch gekennzeichnet, daß** sie eine Reinheit von mehr als 99,8 % G/G, bevorzugt von mehr als 99,9 % G/G hat.

12. Pharmazeutische Zusammensetzung, die die kristalline Base von Citalopram gemäß einem der Ansprüche 1 bis 11 enthält.

13. Pharmazeutische Zusammensetzung gemäß Anspruch 12, die eine Tablette ist, hergestellt durch
a) Direktverpressen von Citalopram, ggf. im Gemisch mit pharmazeutisch akzeptablen Hilfsstoffen;
b) Verpressen eines Naßgranulats des Citaloprams, ggf. im Gemisch mit pharmazeutisch akzeptablen Hilfsstoffen; oder
c) Verpressen eines Schmelzgranulats des Citaloprams, ggf. im Gemisch mit pharmazeutisch akzeptablen Hilfsstoffen.

14. Pharmazeutische Zusammensetzung gemäß Anspruche 12 oder 13, **dadurch gekennzeichnet, daß** sie die racemische Mischung von Citalopram-Base enthält.

## Revendications

1. Base cristalline de citalopram, **caractérisée en ce qu'**elle a une pureté supérieure à 99,8 % en poids, de préférence supérieure à 99,9 % en poids.

2. Base cristalline de citalopram, préparée par un procédé **caractérisé en ce que** l'on libère la forme base du citalopram et on la précipite sous forme cristalline, et on la recristallise éventuellement une ou plusieurs fois.

3. Base cristalline de citalopram selon la revendication 2, **caractérisée en ce que** l'on libère la forme base du citalopram à partir d'un sel brut ou d'un mélange brut de citalopram.

4. Base cristalline de citalopram, préparée par un procédé **caractérisé en ce que** l'on élimine d'un mélange brut de citalopram ou d'un sel brut de citalopram, une ou plusieurs impuretés de formule : 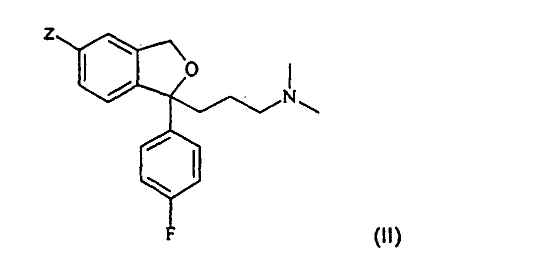 dans laquelle Z est un groupe halogéno, -O-SO₂-(CF₂)ₙ-CF₃ dans lequel n est un nombre de 0 à 8, -CHO, -NHR¹, -COOR², -CONR²R³, où R² et R³ sont choisis parmi l'hydrogène, un groupe alkyle, un groupe aryle ou aralkyle éventuellement substitué, et R¹ est un hydrogène ou un groupe alkylcarbonyle,
en précipitant le citalopram base sous forme cristalline, et éventuellement en recristallisant cette base une ou plusieurs fois.

5. Base cristalline de citalopram selon la revendication 4, dans laquelle on prépare le mélange brut de citalopram contenant le composé de formule II comme impureté en soumettant un composé de formule II à une réaction d'échange de cyanure avec une source de cyanure.

6. Base cristalline de citalopram selon la revendication 4, dans laquelle Z est un halogène, en particulier le chlore ou le brome.

7. Base cristalline de citalopram selon l'une des revendications 4 à 6, dans laquelle on soumet le mélange brut de citalopram à une purification initiale avant de précipiter le citalopram base sous forme cristalline.

8. Base cristalline de citalopram selon l'une des revendications 4 à 6, dans laquelle on soumet le mélange brut de citalopram à une purification initiale avant de former un sel brut à partir dudit mélange brut.

9. Base cristalline de citalopram selon l'une des revendications 4 à 7, dans laquelle on libère le citalopram base à partir d'un sel brut ou d'un mélange brut de citalopram par traitement avec une base et on le soumet éventuellement à une purification supplémentaire avant de précipiter le citalopram base sous forme cristalline.

10. Base cristalline de citalopram selon l'une quelconque des revendications 2 à 4, **caractérisée en ce que** le sel brut est le bromhydrate, le chlorhydrate, le sulfate, l'oxalate, le phosphate ou le nitrate, de préférence le sulfate, le bromhydrate ou le chlorhydrate.

11. Base cristalline de citalopram selon l'une quelconque des revendications 2 à 10, **caractérisée en ce qu'**elle a une pureté supérieure à 99,8 % en poids, de préférence supérieure à 99,9 % en poids.

12. Composition pharmaceutique contenant la base cristalline de citalopram selon l'une des revendications 1 à 11.

13. Composition pharmaceutique selon la revendication 12, qui est un comprimé préparé par :
a) compression directe de citalopram, éventuellement en mélange avec des adjuvants pharmaceutiquement acceptables;
b) compression d'un granulé humide de citalopram, éventuellement en mélange avec des adjuvants pharmaceutiquement acceptables ; ou
c) compression d'un granulé fondu de citalopram, éventuellement en mélange avec des adjuvants pharmaceutiquement acceptables.

14. Composition pharmaceutique selon la revendication 12 ou 13, **caractérisée en ce qu'**elle contient le mélange racémique de citalopram base.
